# EUROPEAN PATENT APPLICATION

(11) **EP 2 818 244 A1**
(43) Date of publication of application: **31.12.2014**
(21) Application number: 13173937.7
(22) Date of filing: 27.06.2013
(51) Int. Cl.: B01L 3/00, G01N 33/50

(54) **Flow chamber and uses thereof**

(71) Applicant: Ospedale San Raffaele S.r.l., 20132 Milano (IT); Politecnico di Milano, 20133 Milano (IT)
(72) Inventor: Pardi, Ruggero, 20132 Milano (IT); Molteni, Raffaella, 20132 Milano (IT); Dubini, Gabriele, 20133 Milano (IT); Bianchi, Elena, 20133 Milano (IT); Lagana', Katia, 20133 Milano (IT)
(74) Representative: Capasso, Olga

(57) **Abstract**

The present invention relates to a flow chamber, relative device and uses thereof. In particular the flow chamber of the invention allows to measure cell adhesion and/or transmigration and/or migration in an environment that reproduces *in vivo* conditions.

## Description

### Technical field

The present invention relates to a flow chamber, relative device and uses thereof. In particular the flow chamber of the invention allows to measure cell adhesion and/or transendothelial migration and/or interstitial migration in an environment that reproduces *in vivo* conditions.

### Background art

Leukocyte extravasation and interstitial migration are interdependent events that play crucial roles in the inflammatory response to injury or infection and contribute, if deregulated, to the pathogenesis of many chronic immune disorders. As essential components of leukocyte trafficking in inflammation, such key processes entail sequential, interrelated steps that regulate the efficacy of the immune response (Molteni, Fabbri et al. 2006; Ley, Laudanna et al. 2007). Leukocyte extravasation initiates with capture of free-flowing leukocytes and subsequent rolling onto the vascular endothelium, primarily mediated by selectins (McEver 2002). These events precede and facilitate leukocyte recognition of chemokines immobilized on the endothelial apical surface leading to integrin-dependent firm adhesion. (Constantin, Majeed et al. 2000; Grabovsky, Feigelson et al. 2000; Hynes 2002; Laudanna, Kim et al. 2002; Kinashi 2005). Further stabilization of the active integrin conformation results in adhesion strengthening to the endothelium, which allows firmly adherent leukocytes to resist hemodynamic shear forces (Molteni, Crespo et al. 2009). Shear-resistant leukocytes can crawl along and eventually migrate across endothelial cells (diapedesis or transendothelial migration), either at intercellular junctions (paracellular pathway), or through the endothelial cell body (transcellular route) (Muller 2009). Therefore, shear forces and biochemical signals relayed by chemokine and adhesive molecules' engagement cooperate to regulate cell adhesion and motility during the extravasation process (Molteni, Fabbri et al. 2006; Ley, Laudanna et al. 2007; Alon and Ley 2008). After leaving the vascular region, leukocytes migrate through the interstitial tissue to approach the site of inflammation. In line with evidence providing that each of the above steps prepares the leukocytes for the subsequent one (Ley, Laudanna et al. 2007; Kolaczkowska and Kubes 2013), emerging data support a role for transendothelial migration in regulating the behaviour and cellular functions of leukocytes in the extravascular tissue (Nourshargh, Hordijk et al. 2010). There is evidence that transmigrating neutrophils exert a mechanical force onto the underlying substratum that may disrupt endothelial cell-cell contacts and influence penetration of the basement membrane (Rabodzey, Alcaide et al. 2008). Moreover, recent data indicate that migration through endothelial cell junctions mediates changes in leukocyte integrin and protease expression and/or activation, which might increase the ability of leukocytes to interact with and penetrate the perivascular basement membrane. In turn, interaction with the basement membrane could facilitate leukocyte motility in the extracellular matrix and enhance cellular responses, crucial for an effective inflammatory response (Nourshargh and Marelli-Berg 2005; Ley, Laudanna et al. 2007).

Spatiotemporal analysis of such leukocyte trafficking towards inflammatory sites has been limited by difficulties of proper reconstitution of inflammation *in vitro.* Indeed, although a number of *in vitro* assays have increased the knowledge about the mechanisms of leukocyte recruitment, conventional devices address only specific events and fail to recapitulate the whole process in a *in vivo*-like milieu (Bianchi, Molteni et al. 2013). Expressly, a variety of established microfluidic-based technologies allow to monitor the first key sequence of events occurring in the vascular compartment, the multistep extravasation process, but are unable to address the migratory cell responses in the extravascular tissues. Indeed, the parallel-plate flow chamber (Brown and Larson 2001; Lawson, Rose et al. 2010) and the capillary flow chamber with a cylindrical configuration (Cooke, Usami et al. 1993) are engineered to specifically investigate leukocyte behaviour onto adhesive substrates (adhesion molecules/endothelial cells) and, if possible, diapedesis under controlled fluid dynamic conditions. Moreover, the Boyden chamber (Boyden 1962), traditional device developed for shear-free cell chemotaxis/transmigration assays, has been implemented in a transwell flow chamber for monitoring leukocyte transendothelial migration under flow (Schreiber, Shinder et al. 2007). Further development of such technologies led to an advanced transwell flow chamber where endothelial and stromal cells are co-cultured onto two opposite sides of a porous filter to better mimicking the *in vivo* vascular conditions (McGettrick, Filer et al. 2007). Differently, other conventional devices address the specific ability of cells to migrate towards a gradient of chemokines within two-dimensional environments, typically occurring during leukocyte crawling along the vascular endothelium. They include the Dunn chamber (Zicha, Dunn et al. 1991) and the Zigmond chamber (Zigmond 1977), where cells migrate on a glass coverslip guided by a diffusive gradient across a bridge between two wells, the under-agarose assay (Nelson, Quie et al. 1975), measuring cells migrating on a glass coverslip underneath an agarose gel across which a gradient is formed, the micropipette set up (Gerisch and Keller 1981), releasing chemoattractants to create gradients and microfabricated devices able to generate stable, linear chemokine gradients (Li Jeon, Baskaran et al. 2002; Irimia, Liu et al. 2006). Finally, more recent solutions allow to assess cell interstitial migration within a three-dimensional (3D) microenvironment, as it occurs during extravascular leukocyte migration, but fail to recapitulate the upstream vascular events. Basic collagen-based models have been established to examine leukocyte motility in a tissue-like context (Gunzer, Schafer et al. 2000; Wolf, Alexander et al. 2009; Sixt and Lammermann 2011). More recent microfluidic devices have introduced the possibility to expose cells embedded in a 3D extracellular matrix to more controlled and/or stable chemotactic gradients (Saadi, Rhee et al. 2007; Abhyankar, Toepke et al. 2008; Haessler, Kalinin et al. 2009; Sackmann, Berthier et al. 2012). Additionally, an advanced microfluidic tool has been engineered to model in a shear-free microenvironment leukocyte diapedesis across an endothelial layer and subsequent interstitial cell migration (Han, Yan et al. 2012).

WO 2006/105329 relates to a device for parsing out a multi-phased biological event, the device comprising: (a) a housing further comprising: (i) a chamber; (ii) an inlet connected to the chamber for delivering a substance into the chamber; (iii) an outlet connected to the chamber for removing a substance from the chamber; (iv) at least one membrane, wherein the membrane is situated within the chamber of the housing such that at least two compartments are created by the relation of membrane surfaces with the chamber; (b) a delivery means; and (c) a receiving means, wherein a substance is delivered into the chamber of the housing via the inlet and constituents of the substance engage in various phases of a multi-phase biologic process.

The document also relates to a method for separating the multiple phases of HSPC homing using the above device and to a method for analyzing the phases of HSPC homing comprising incubating a membrane of the device with an endothelial cell line. However, this device does not correctly mimics the inflammatory microenvironment and does not recapitulate the whole process of leukocyte recruitment into the inflammatory site.

WO 2007/035301 relates to compositions and methods for preparation of a three-dimensional transendothelial cell migration (TEM) assay. The composition for detecting migration of cells comprises a solid layer comprising collagen gel; a first cellular layer in contact with the solid layer and comprising a first cell type; and a second cell type seeded on top of the first cellular layer. A 96 well plate format is described, the combination with a high throughput cellular scanner enables high throughput TEM assay. In the system of this document cells are not properly mechanically stimulated by a flow, as they should be in an *in vivo* context.

### Summary of the invention

Leukocyte extravasation and interstitial migration are key events in the inflammatory response to injury or infection. Traditional *in vitro* techniques address only specific adhesive/migratory steps of leukocyte recruitment to inflamed tissues and fail to recapitulate the whole process in a proper three-dimensional (3D) microenvironment. The device the authors developed provides a real-time method to investigate the interdependence between sequential steps underlying leukocyte trafficking in a close-to-physiology *in vitro* context. The authors' flow chamber consists of an endothelium-coated filter laid over a 3D extracellular matrix where chemoattractants can form a gradient. Differentially treated and labeled leukocytes, flowing onto endothelial cells, can be compared for their ability to establish adhesive interactions, transmigrate and migrate interstitially. Quantitative analysis of leukocyte behaviour revealed that in the presence of a chemotactic gradient, neutrophils increase their migration velocity, directionality and chemotactic efficiency through the extracellular matrix. The technology of the present invention offers new and versatile tools to thoroughly investigate the stepwise process of leukocyte recruitment into inflamed tissues *in vitro.*

The microfluidic device of the present invention overcomes the limited, step-specific analysis of traditional *in vitro* assays by providing a comprehensive method for *in vitro* modeling the whole process of leukocyte recruitment to inflammatory sites. The platform allows i) to reproducibly recapitulate in an *in vivo*-like microenvironment the interdependent steps underlying leukocyte extravasation and interstitial migration; ii) to perform high-resolution, real-time 2D and 3D imaging of leukocyte extravasation and interstitial migration respectively; iii) to concurrently compare the behaviour of multiple sets of differentially labeled leukocytes. Image processing and data analysis are then performed. The possibility to modulate microenvironment-related parameters, including shear flow and extracellular matrix composition, makes the microfluidic chamber of the invention highly versatile. A method for deeply and reliably understanding the mechanisms of leukocyte recruitment is then provided and might aid the identification and validation of novel anti-inflammatory drug targets. The authors exploited the potential of the above tool by investigating neutrophil trafficking in response to the inflammatory Keratinocyte-derived chemokine (KC).

It is therefore an object of the present invention a flow chamber for cell assay essentially comprising :
- a bottom holder (3) and a top holder (2), both holders having each one at least one
   opening, the bottom and the top holder being sealable to each other;
   said holders containing from the top to the bottom:
- a lid (4) comprising two connections (15a) and (15b), passing through the at least one opening of the top holder (2), one connectable to an inlet port (1) and the other connectable to an outlet port (11);
- a first gasket (10) comprising a first channel (14), each far end of the channel (14) being aligned with the connections (15a) and (15b), respectively;
- a filter (9), optionally coated with cells;
- a second gasket (8) comprising a second channel (18) being aligned with the first channel (14) and having at least the same area as said first channel (14);
- a slide (7), so that the second channel (18) and the slide (7) define a chamber (20) being filled with at least one layer of gel.

Preferably the top holder (2) and the lid (4) are assembled in one piece.

In a preferred embodiment the cells on filter (9) are endothelial cells. Preferably the endothelial cells are stimulated with a pro-inflammatory agent, preferably TNF or IL-1.

Still preferably the endothelial cells are coated with an agent that increases cell arrest and transmigration across endothelial cells. Preferably the agent is a chemoattractant, preferably the chemoattractant is selected from the group consisting of: formyl peptides, chemokines (mostly CC or CXC chemokines), complement 3a (C3a) and complement (C5a), leukotrienes.

In a preferred embodiment the chamber (20) is filled with a first and a second layer of gel, the second layer of gel optionally comprising an agent that increases cell motility, preferably a chemoattractant, more preferably the chemoattractant is selected from the group consisting of: formyl peptides, chemokines (mostly CC or CXC chemokines), complement 3a (C3a) and complement (C5a), leukotrienes.

It is a further object of the invention a device for measuring cell adhesion and/or transendothelial migration and/or interstitial migration comprising the flow chamber as described above and a flow producing means connected thereto through the inlet port (1).

It is a further object of the invention the use of the flow chamber as described above or of the device as described above to assess adhesion and/or transendothelial migration and/or interstitial migration of cells introduced by the inlet port (1).

It is a further object of the invention a method to assess cell adhesion to endothelial cells by means of the flow chamber of the invention or the device as described above comprising the steps of:
- introducing a cell suspension through the inlet port (1) and allowing the cells to flow through the first connector (15a), the channel (14) and to adhere to the filter (9) coated with endothelial cells;
- measuring with appropriate imaging means the adhesion of cells to endothelial cells.

It a is a further object of the invention a method to assess cell transmigration across endothelial cells by means of the flow chamber as described above or the device as described above comprising the steps of:
- introducing a cell suspension through the inlet port (1) and allowing the cells to flow through the first connector (15a), the channel (14), to adhere to and migrate across the filter (9) coated with endothelial cells to reach the chamber (20);
- measuring with appropriate imaging means the transendothelial migration.

It is a further object of the invention a method to assess cell interstitial migration by means of the flow chamber as described above or the device as described above comprising the steps of:
- introducing a cell suspension through the inlet port (1) and allowing the cells to flow through the first connector 15(a), the channel (14), to adhere to and migrate across the filter (9) optionally coated with endothelial cells into the chamber (20) filled with the first and second gel layers, to migrate through the first gel layer, laid over the second one, said second gel layer optionally comprising an agent that increases cell motility;
- measuring with appropriate imaging means the interstitial migration of cells.

It is a further object of the invention a method to assess cell adhesion to endothelial cells, cell transmigration across endothelial cells and cell interstitial migration or cell adhesion to and transmigration across endothelial cells or cell transmigration across endothelial cells and interstitial migration by means of the flow chamber or the device of the invention comprising the step of:
- introducing a cell suspension through the inlet port (1) and allowing the cells to flow through the first connector (15a), the channel (14), to adhere to and migrate across the filter (9) coated with endothelial cells into the chamber (20) filled the first and second gel layers, and to migrate through the first gel layer, laid over the second one, said second gel layer optionally comprising an agent that increases cell motility;
- measuring with appropriate imaging means the adhesion of cells to endothelial cells, cell transmigration across endothelial cells and the interstitial migration of cells.

Preferably the cell suspension is a leukocyte suspension, a tumor cell suspension or a stem cell suspension.

It is a further object of the invention a method to identify an agent inhibiting extravasation and/or interstitial migration comprising using the flow chamber or the device as described above in the presence or absence of a test agent, wherein if decreased migration and/or adhesion is observed in the presence of the test agent when compared to migration and/or adhesion in absence of the test agent, then the inhibitory agent is identified.

Preferably the lid (4) has a thickness between 1 mm and 1 cm.

Preferably the first gasket (10) has a thickness between 70 um and 300 um, preferably 200 um. Preferably the second gasket (8) has a thickness between 250 um and 500 um, preferably 500 um.

Preferably the first gasket (10) and/or the second gasket (8) are flexible.

Preferably the first gasket (10) and/or the second gasket (8) are made of silicone rubber.

Preferably the channel (14) and the channel (18) have rectangular shape. Preferably the height is from about 70 um to about 300 um (for cells of 7 - 15 um in diameter), preferably the width is at least about 10 times the height, so preferably 700 um to 3000 um, in order to generate the correct flow for the mechanical stimulation of the cells. Preferably the length of the channel, considering only the part in between the two connections (15a) and (15b), has to be between 1 and 3 centimeters.

Preferably the diameter of filter (9) pores are between 3 and 8 um.

Preferably the filter (9) has a thickness of about 15 um.

Preferably the slide (7) is of transparent material.

Preferably the slide (7) is of 100-200 um thickness.

Preferably the first layer of gel is a matrix allowing cell migration.

Preferably the first layer of porous gel matrix is a collagen I-based matrix, preferably with a defined composition (i.e. containing extracellular matrix proteins and proteoglycans).

Preferably the second layer of gel, if present, may optionally comprise an agent that increases cell motility (i.e a chemoattractant).

Preferably the second layer of gel is an agarose gel or an inert matrix, transparent to fluorescence, able to provide a mechanical support to the extracellular matrix placed on top of it and unable to bind the chemoattractant which has to diffuse into adjacent layers. The chamber (20) can be filled with additional layers of gel.

Preferably the slide has a thickness between 100 and 200 um.

Preferably the method of the invention allows to assess cell recruitment into an inflammatory site.

Preferably the method of the invention allows to assess leukocyte extravasation and/or interstitial migration.

The invention will now be illustrated by means on non-limiting examples in reference to the following figures.

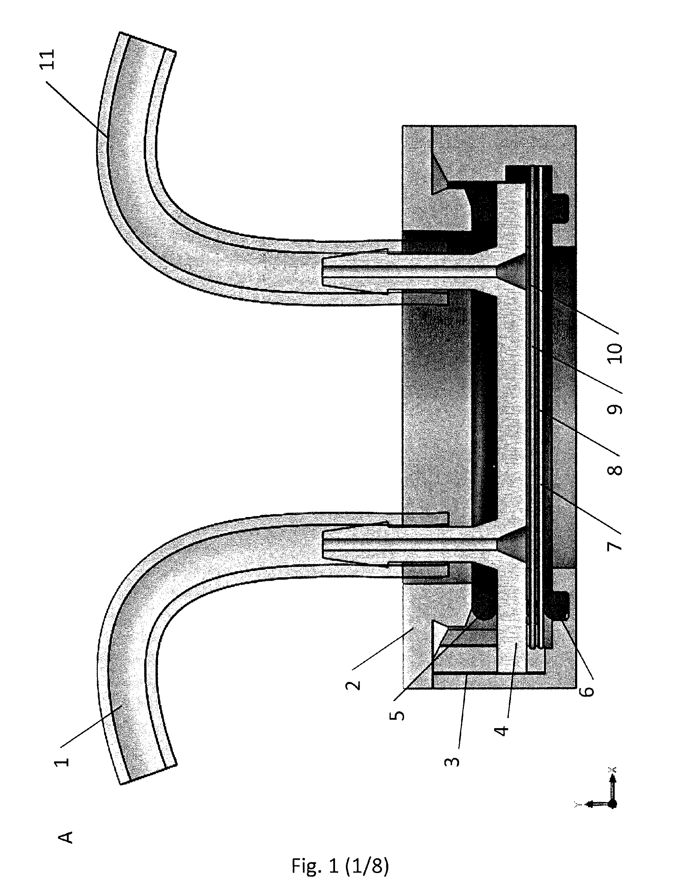

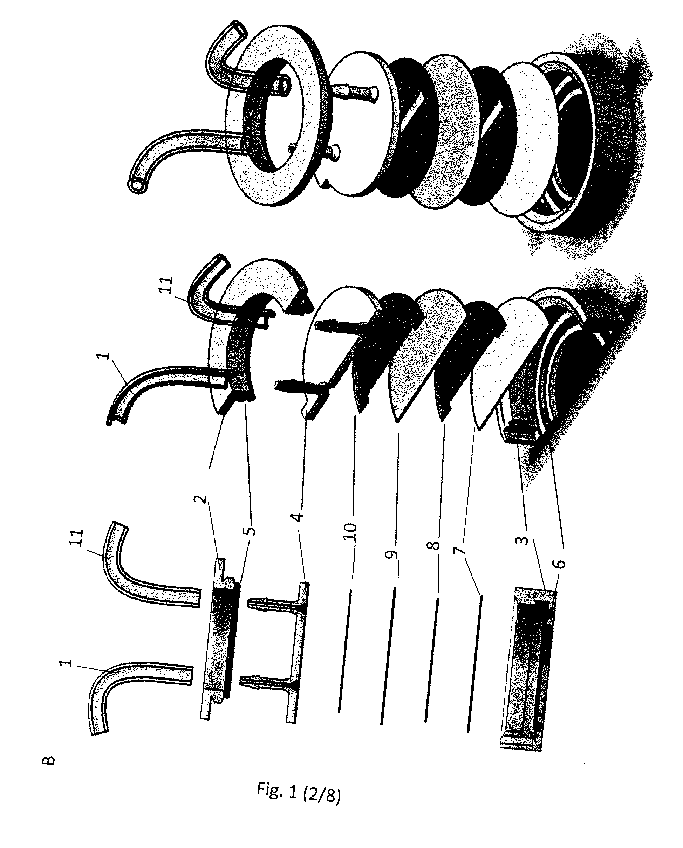

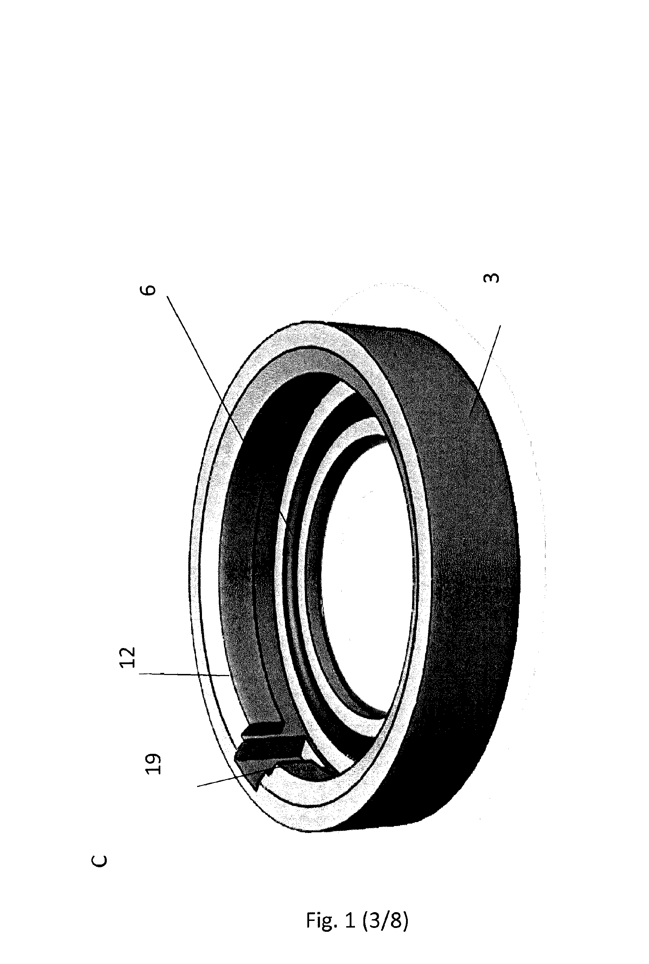

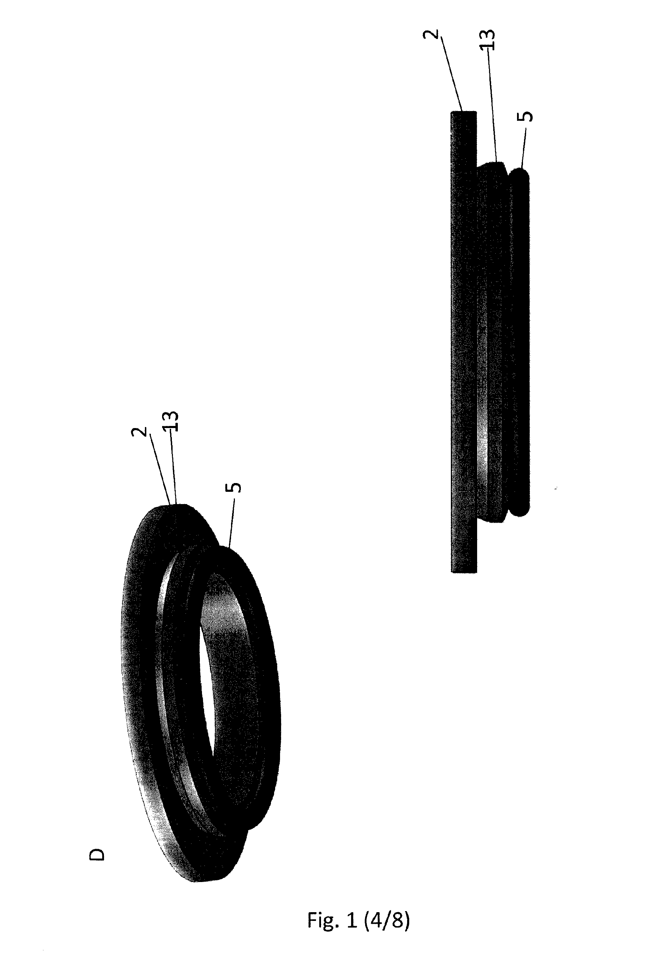

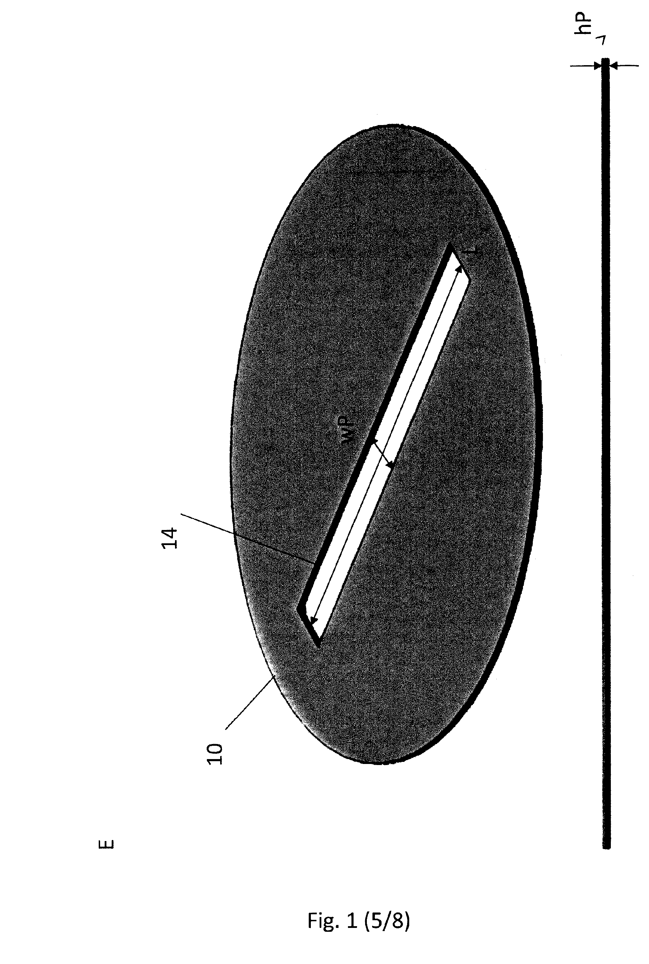

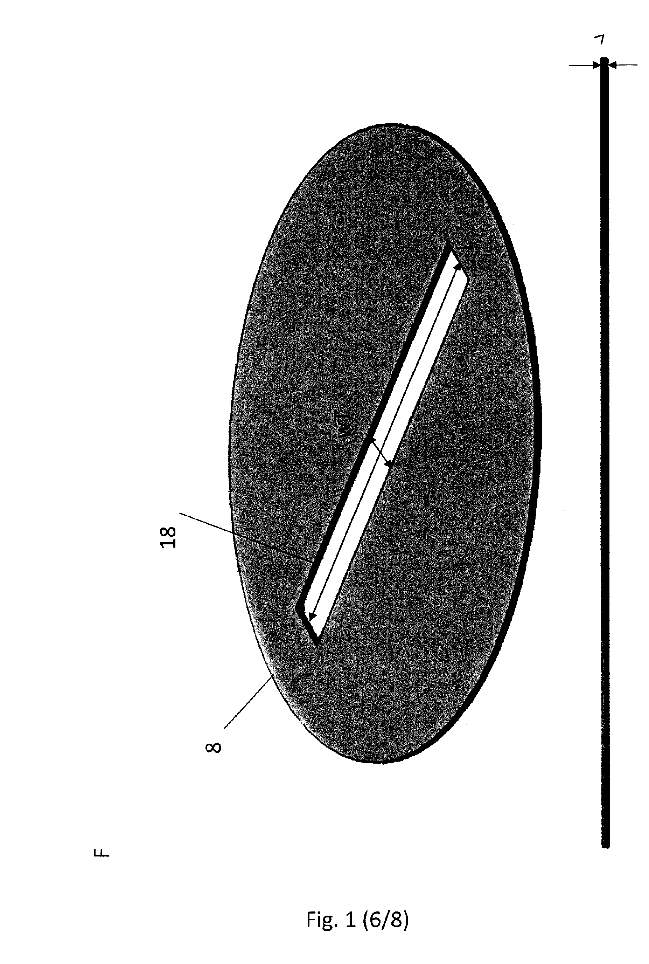

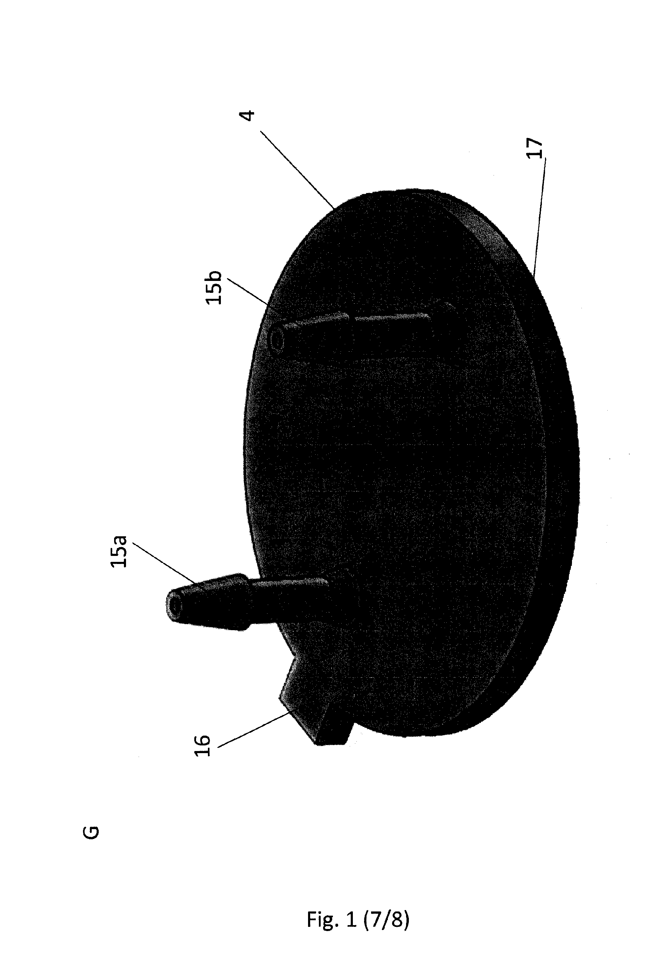

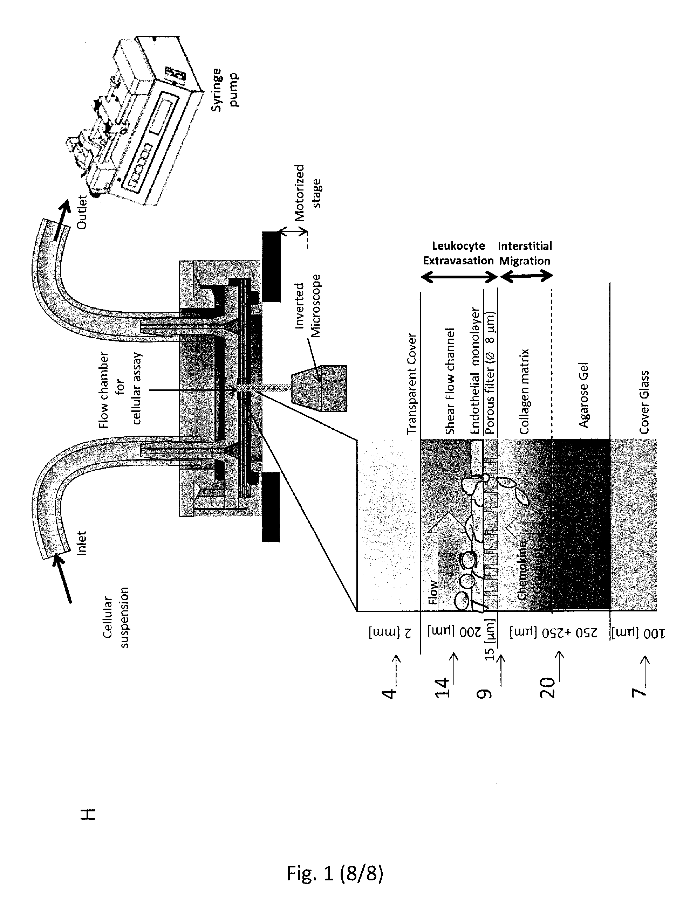

**Figure 1****. Microfluidic Device design: a)** Schematic view of the device. **b)** Schematic view of the layers. **c)** Bottom component. **d)** Top component. **e)** Gapped gasket (200 um thick) characterized by a flow section (wp * hp) and a length L. **f)** Gapped gasket (500 um thick) characterized by a gap (wt * ht *L). **g)** lid. **h)** Experimental setup

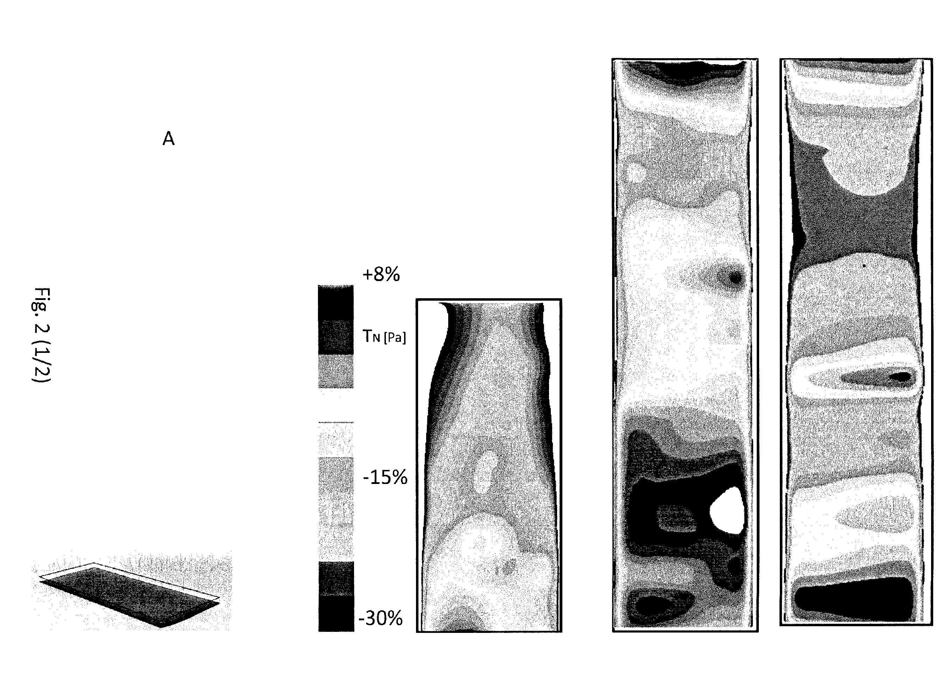

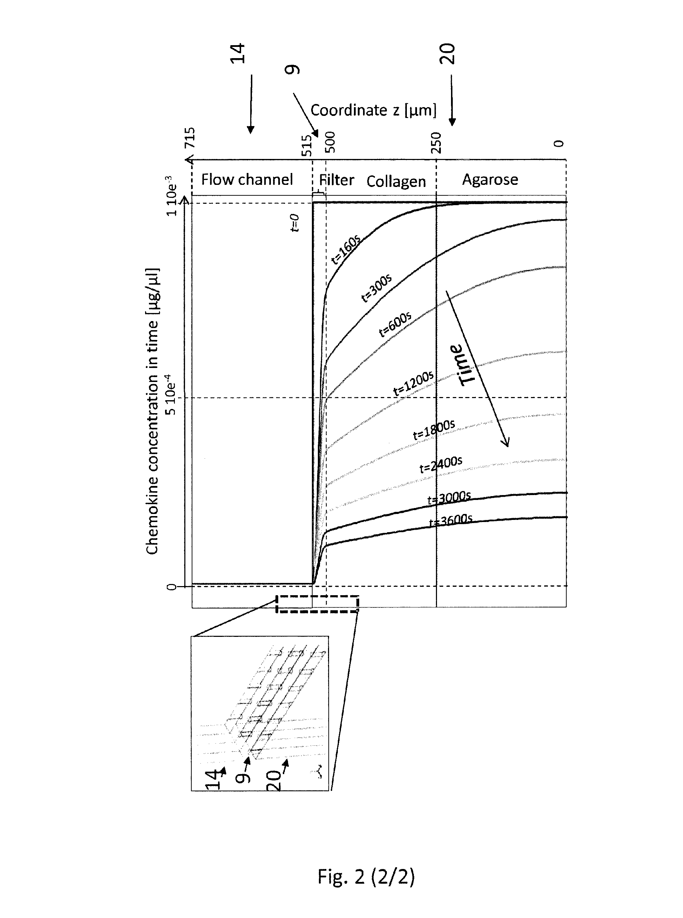

**Figure 2****. Technical Validation of the platform: a)** Contour maps, from numerical simulation, of wall shear stress distribution on three reconstructed portions of the porous filter (9). Wall shear stress is generated by fluid flow in the channel (14) on the surface of the filter. TN is the expected analytically estimated value of wall shear stress. **b)** Time changes from numerical simulation of chemokine gradient across the collagen I gel layer, in the presence of fluid flow in the channel (14). In the box a portion of the geometrical model for 3D fluid dynamic computational simulations is shown: (14) is the channel, (9) is the porous filter, (18) is the gel chamber under the filter (9).

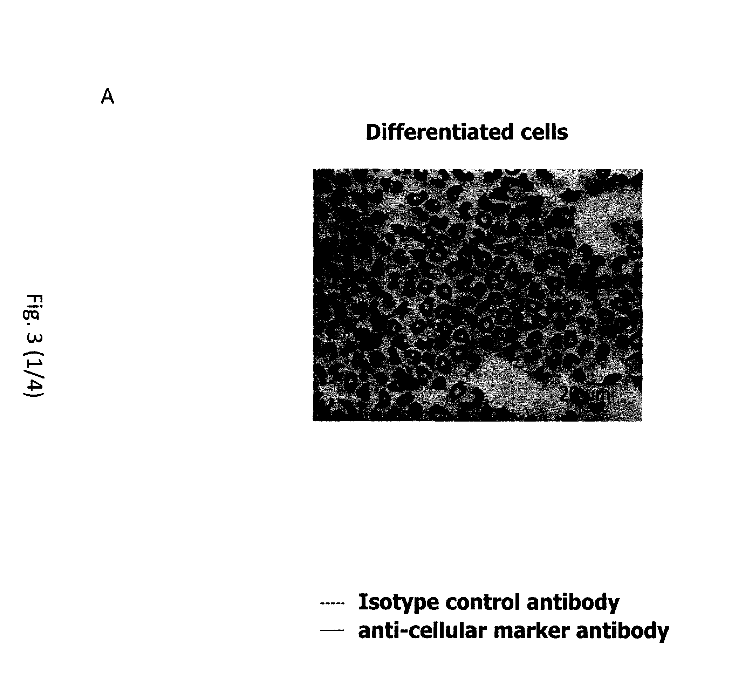

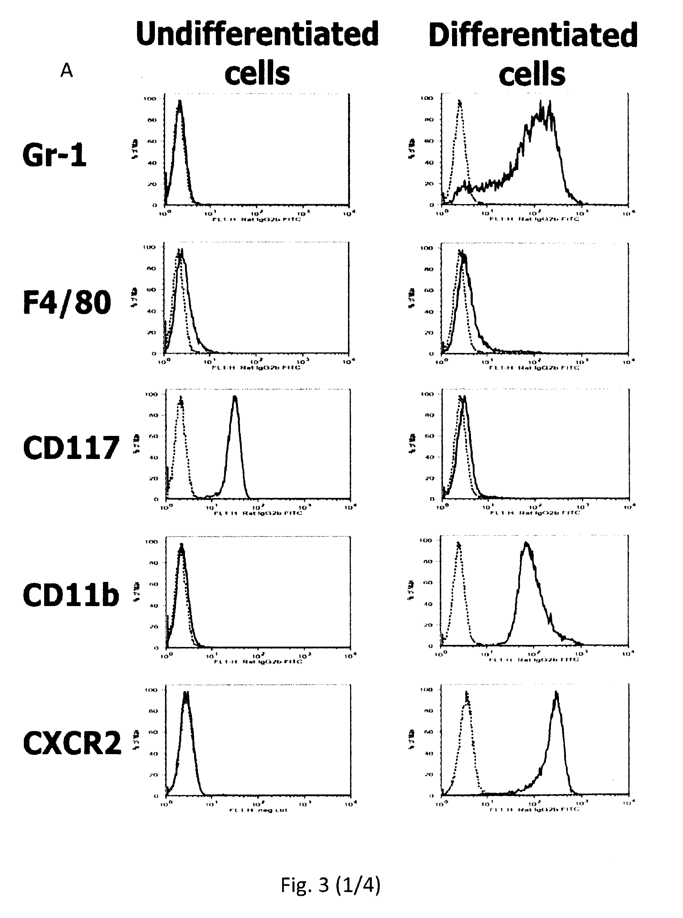

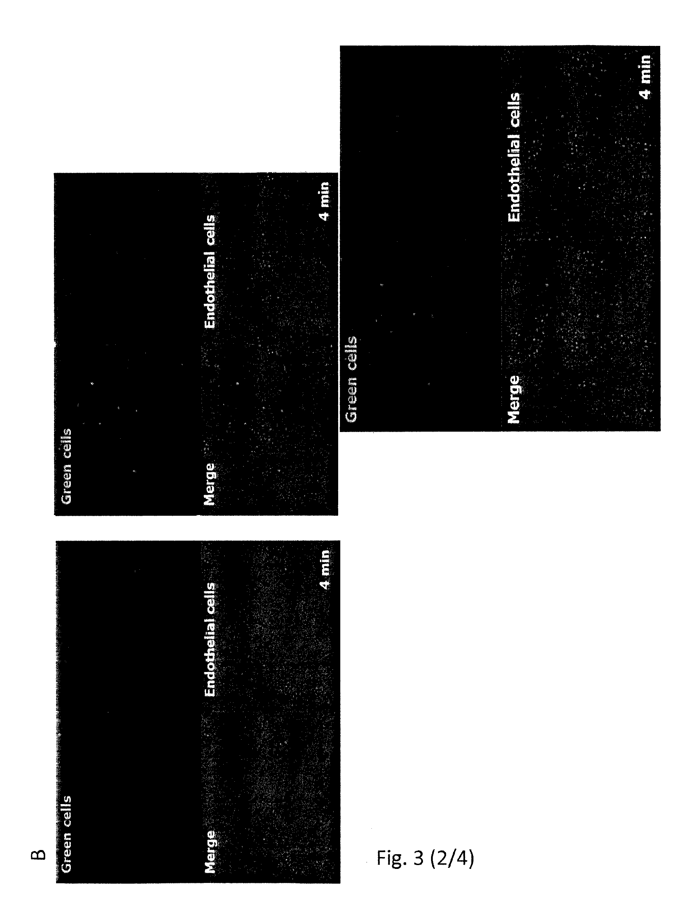

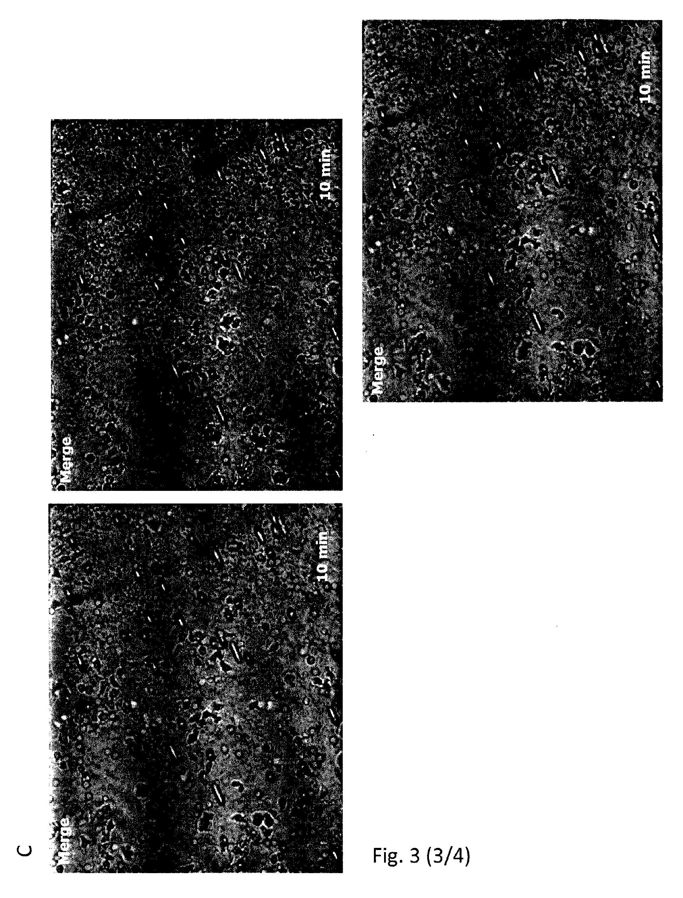

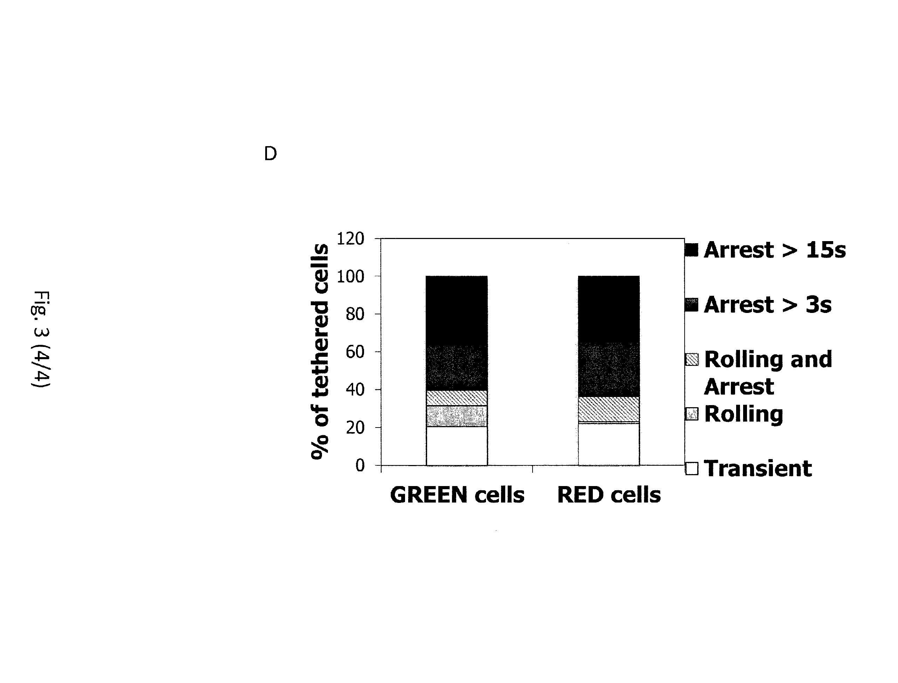

**Figure 3****. Real-time imaging and automated tracking of neutrophil extravasation: a)** Cellular model: murine neutrophils differentiated from immortalized myeloid precursors. Neutrophils stained with the "Diff-Quick" kit and FACS analysis of neutrophil differentiation markers are shown. **b)** The multistep leukocyte adhesion cascade: comparison between wild-type neutrophils, labeled with either Green or Orange CellTracker^{™} dyes, for the ability to tether, roll, arrest onto endothelial cells under shear flow. Phase-contrast images of the endothelium-coated filter taken at regular intervals are also depicted. One representative experiment out of 7 is shown. **c)** Leukocyte transendothelial migration: comparison between wild-type neutrophils, labeled with either Green or Orange CellTracker^{™} dyes, for the ability to migrate across endothelial cells under shear flow. Images of Green/Red leukocytes and endothelial cells taken at regular intervals are shown. One representative experiment out of 7 is shown. **d)** Quantitative analysis of leukocyte-endothelium interactions. Frequencies of adhesive categories are determined as a percentage of cells flowing immediately over the substrate. One representative experiment out of 7 is shown (see the "Material and Methods" section for details).

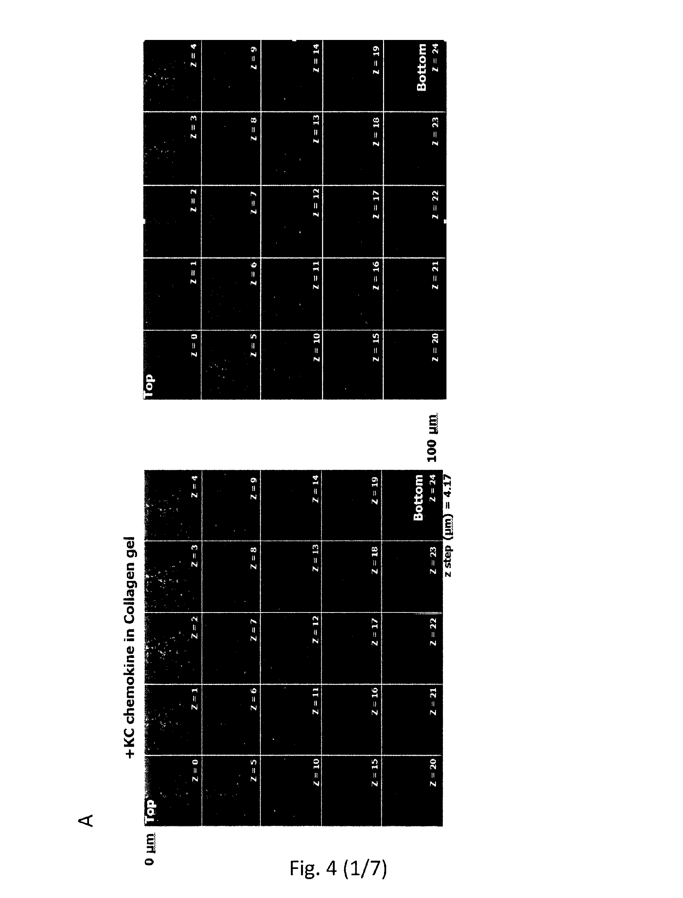

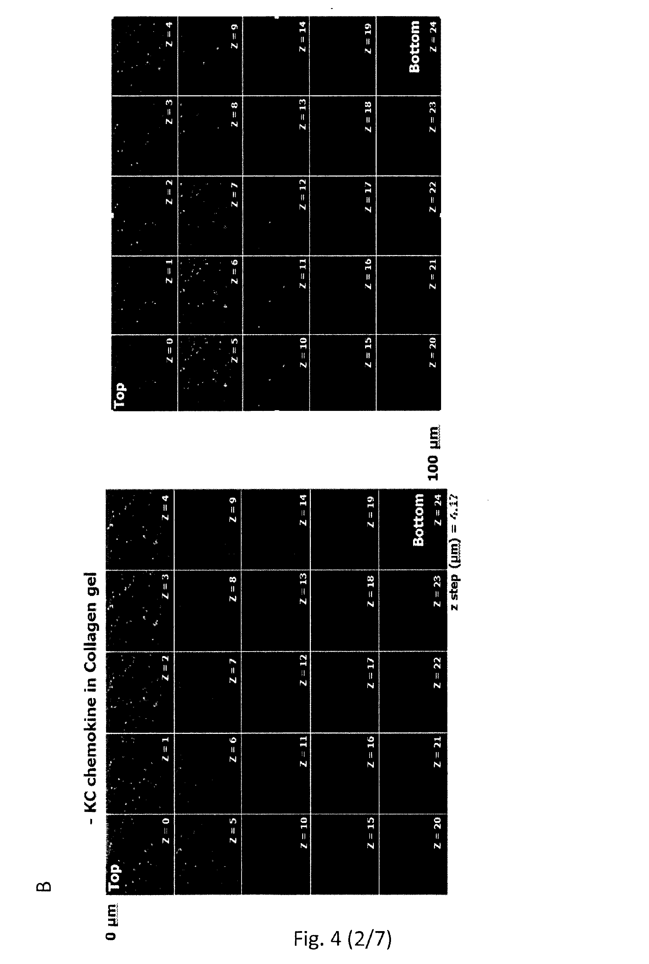

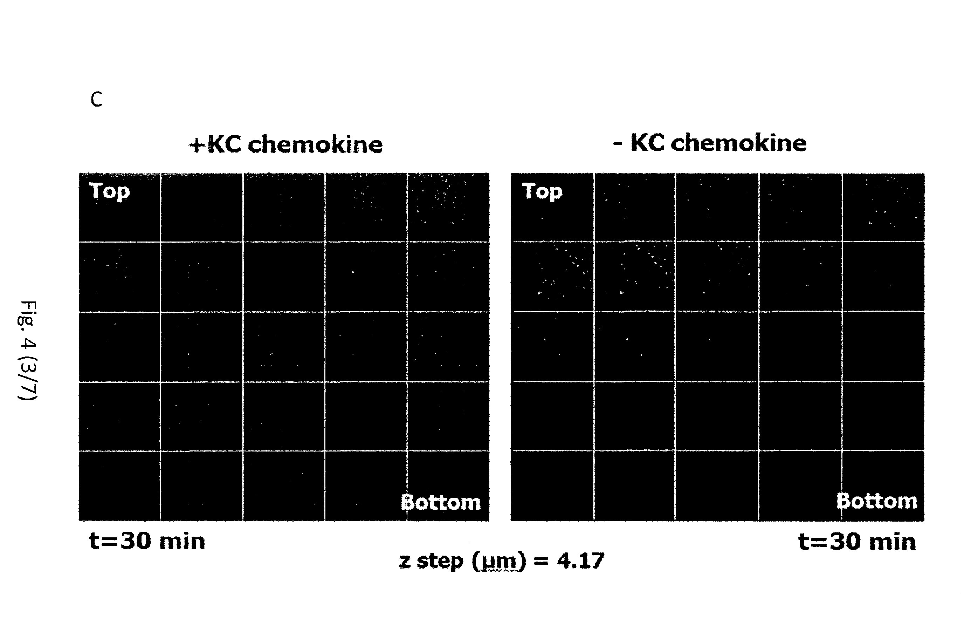

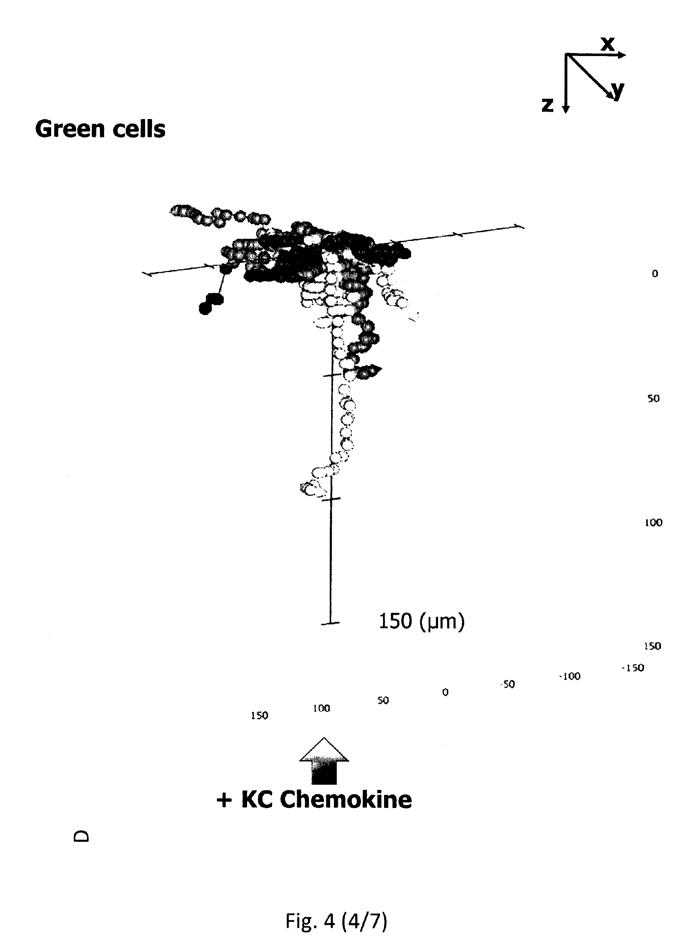

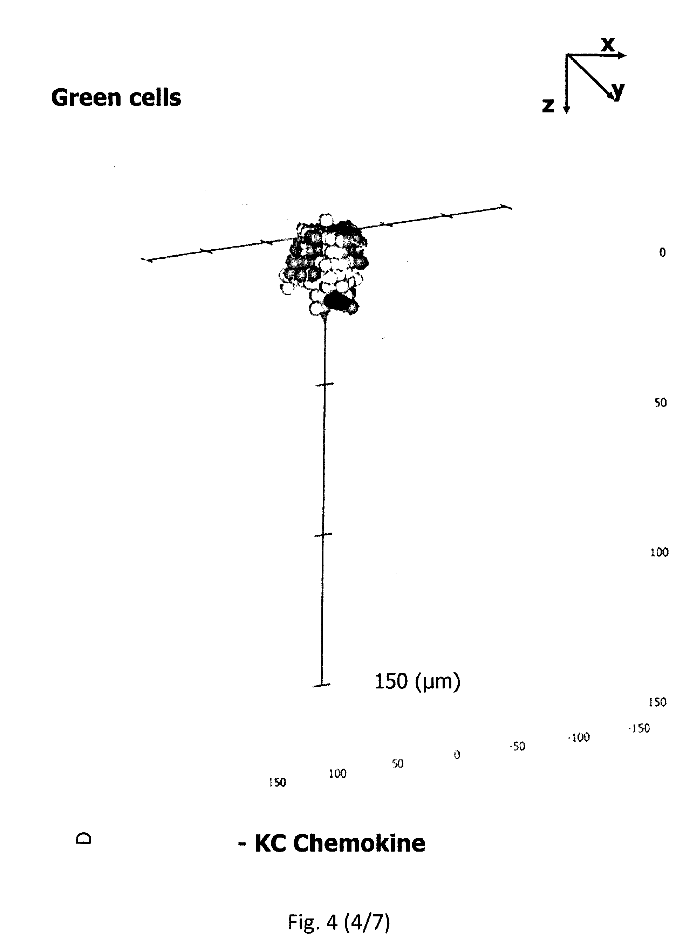

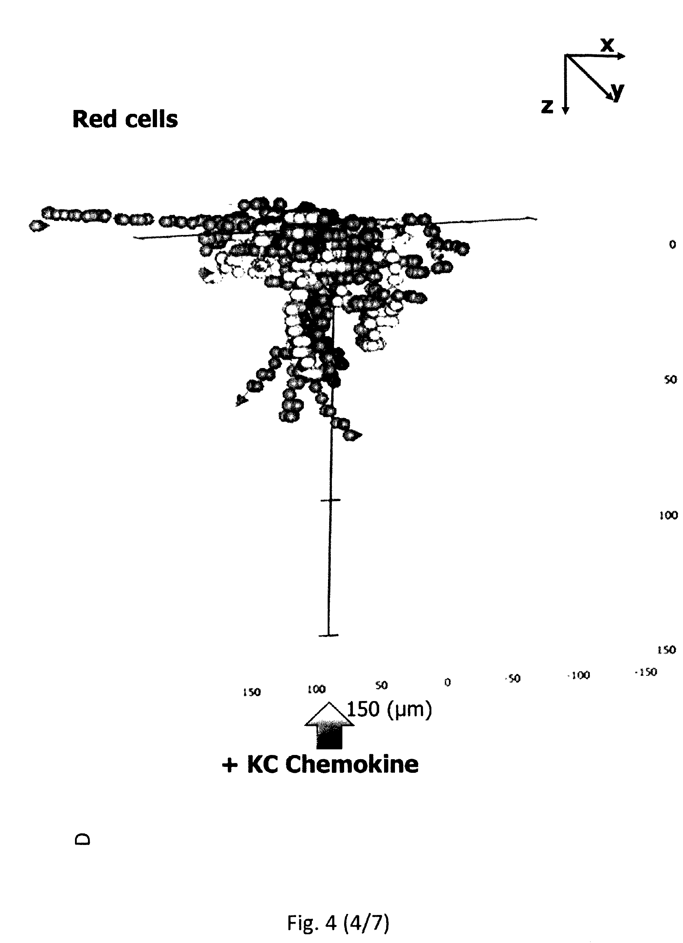

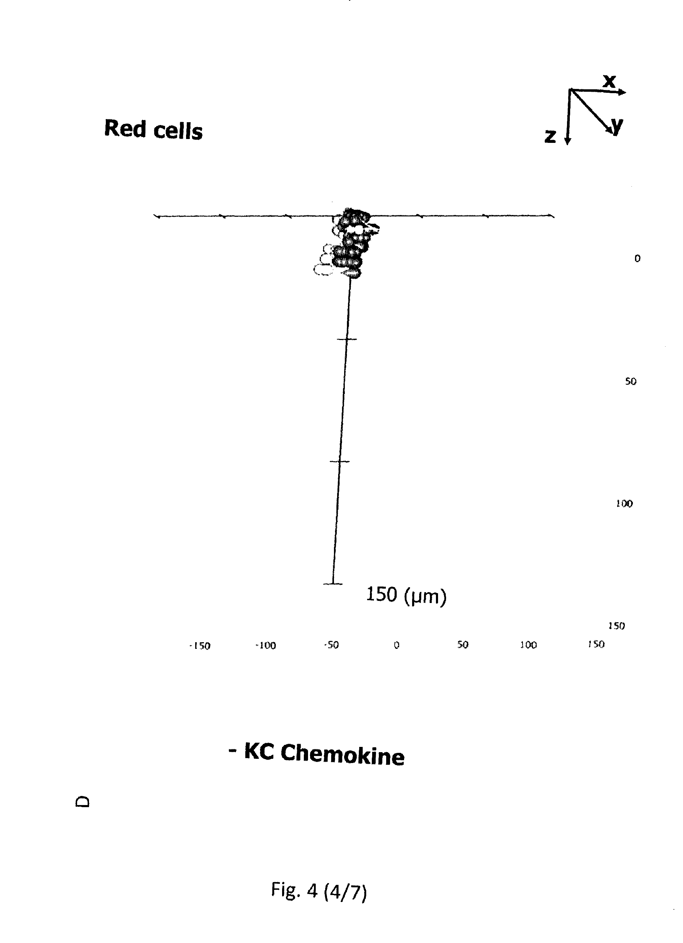

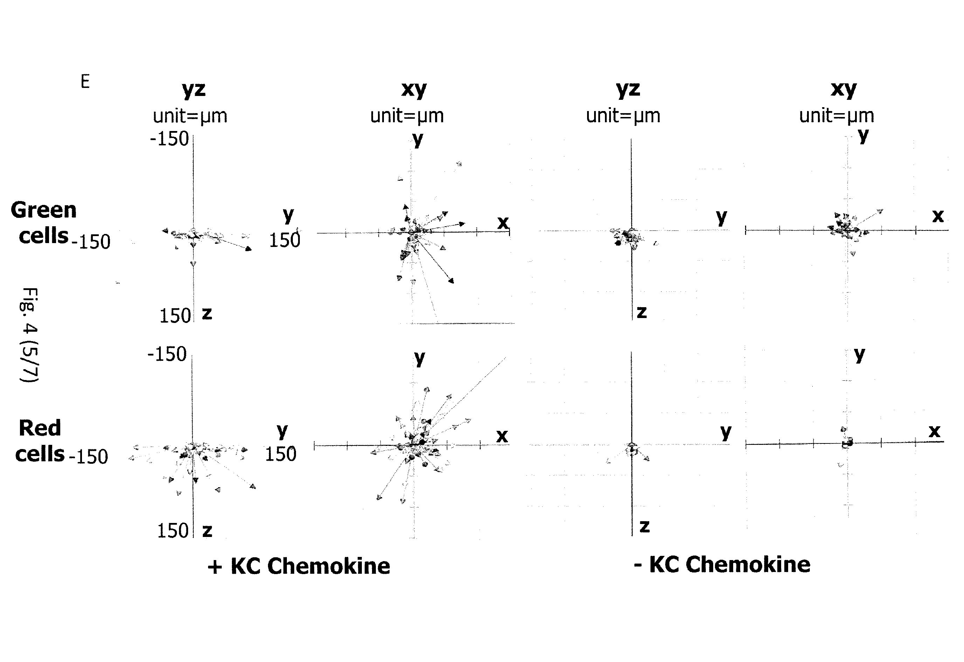

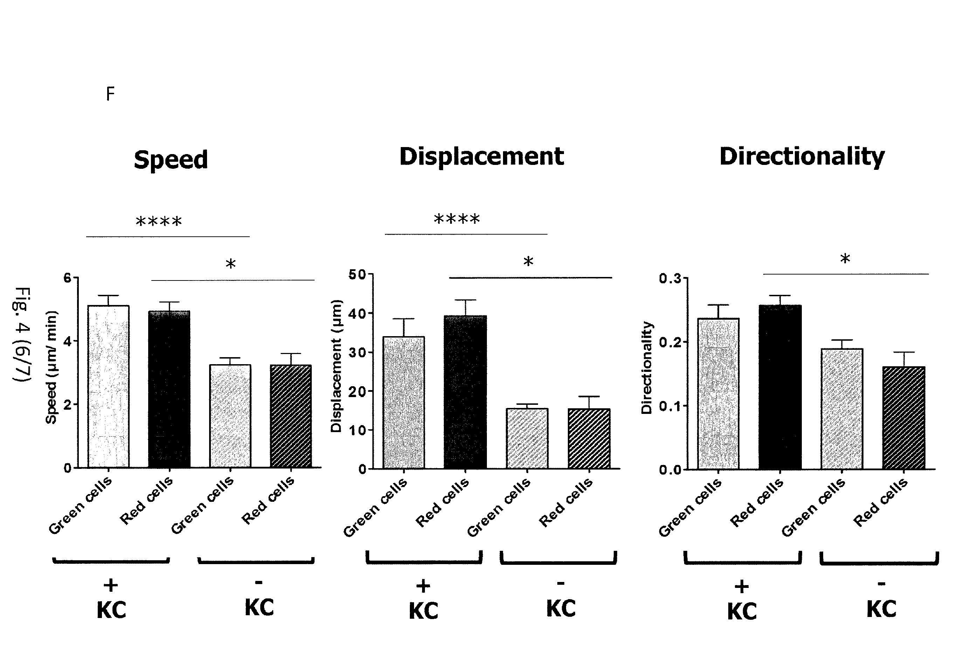

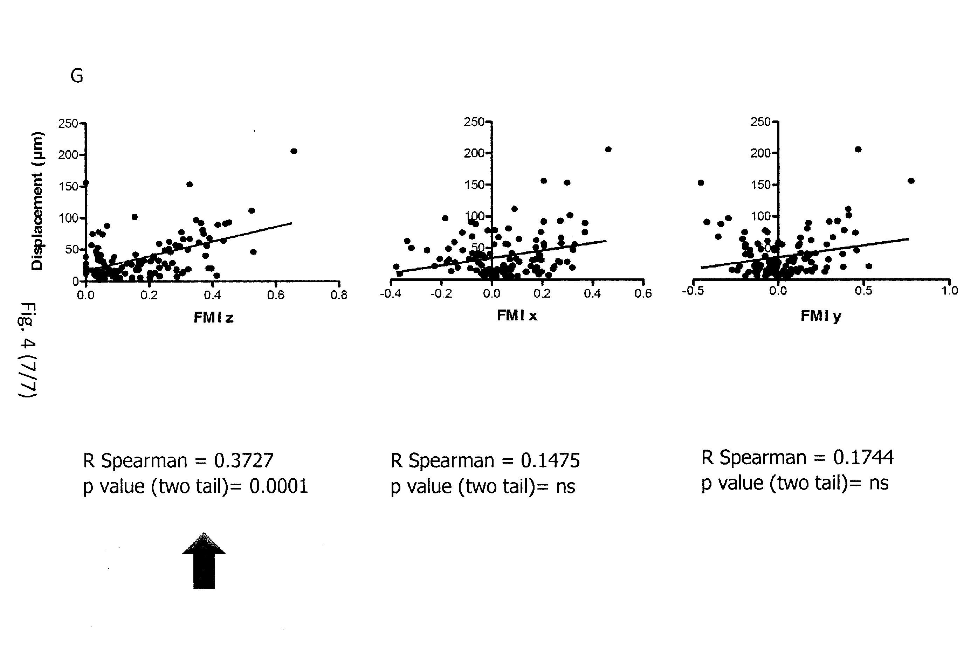

**Figure 4****. Real-time imaging and tracking of neutrophil interstitial migration in the presence or absence of a chemokine gradient within the 3D collagen matrix: a)** Comparison between wild-type neutrophils, labeled with either Green or Orange CellTracker^{™} dyes, for the ability to migrate across the extracellular matrix in the presence of a KC gradient. A time-lapse acquisition of 25 z planes (from z = 0 to z = 24) along the z axis of the collagen matrix (perpendicular to the endothelial layer) is depicted (see the "Material and Methods" section for details). One representative experiment is shown. **b)** Comparison between wild-type neutrophils, labeled with either Green or Orange CellTracker^{™} dyes, for the ability to migrate across the extracellular matrix in the absence of a KC gradient. A time-lapse acquisition of 25 z planes (from z = 0 to z = 24) along the z axis of the collagen matrix is depicted (see the "Material and Methods" section for details). One representative experiment is shown. **c)** Comparison between interstitial migration in presence or absence of a KC gradient. **d)** Quantitative analysis of neutrophil interstitial migration: 3D cells tracks, as determined by manual cell tracking using the Volocity 3D Image Analysis Software. **e)** Quantitative analysis of neutrophil interstitial migration: xyz track vectors, defined as straight lines between cell start and end points . **f)** Quantitative analysis of neutrophil speed, displacement and directionality (see the "Material and Methods" section for details. **g)** Spearman's rank correlation of displacement versus FMI in KC-stimulated cells (see the "Material and Methods" section for details).

### Detailed description of the invention

### MATERIAL AND METHODS

### Device assembly (see Fig. 1b)

The device components are assembled as follows (Fig. 1b):
a) the gapped gasket (8), preferably obtained by two overlapping 250 um-thick gaskets, preferably made of silicone rubber (GlycoTech), is laid over the slide (7), preferably made of glass (VWR International); alternatively the gasket (8) is made of a single part of about 500 um thickness;
b) the slide and the overlapped gasket (7 + 8) are located in the bottom component (3), preferably made of steel, laid on the O-ring (6);
c) 1.2% agarose solution (UltraPure^{™} Agarose 1000; Life Technologies - Invitrogen) is prepared in Hank's Balanced Salt Solution (HBSS; Life Technologies - Invitrogen) without Phenol Red, supplemented with 1 mM Ca²⁺/Mg²⁺, 10 mM Hepes (Gibco - Invitrogen) and 0.1% Bovine Serum Albumin (BSA; Sigma - Aldrich). Upon letting the solution cool at 37°C, KC (2 ug/mL final concentration; R&D Systems) is added; the use of a chemoattractant is optional, its presence increases cellular motility and migration directionality. Any chemoattractant may be used and is chosen depending on the studied cell type and the biological process to be reproduced;
d) the chemoattractant containing agarose solution (40% of the gasket gap volume) is pipetted into the gasket gap (18, Fig. 1f) and is let jellify for few minutes;
e) a collagen solution (1.6 mg/mL final concentration, 60% of the gasket gap volume; preferably collagen I solution, BD Biosciences) is then pipetted over the agarose gel and let jellify for 30 minutes at 37°C. A slight positive meniscus should form when collagen is added; this prevents air bubbles from being trapped when the filter is applied;
f) the endothelium-coated filter (9; Millipore®), pre-coated with 2 ug/mL of KC, is placed over the gasket (8), with the endothelium-coated side facing up; chemokine pre-coating increases recruitment adhesion and transendothelial migration of cells, preferably leukocytes, to the endothelium layer. Any chemoattractant may be used and is chosen depending on the studied cell type and the biological process to be reproduced;
g) the gasket (10), preferably 200 um thickness, preferably made of silicone rubber (GlicoTech) is let to adhere to the lid (4), on the surface (17) aligning the far ends of the channel-shaped gap with the inlet (15a)/outlet (15b) ports;
h) the lid and the adhered gapped gasket (4 + 10) are applied over the underlying layers, aligning the gasket gap (14) with the underlying one (18) containing jellified matrices (Fig. 1e and 1f); the layers are aligned one to the other and kept in place by the coupling of the lid feature (16) to the cavity (19) of the bottom piece (3);
i) the top component (2), preferably made of steel, is screwed on the bottom component (3) by the thread (12) to the thread (13). The above described assembled layers are in contact with the top piece (2) by the o-ring (5);
l) tubing (1 and 11) is connected to fittings (15a) and (15b) of the lid (4), enabling cell suspension, preferably leukocytes, to enter the channel by the inlet tubing (1), drawn through the outlet one (11) connected to a programmable withdrawal syringe pump.

In such a platform, cells are allowed to: i) flow onto endothelial cells under a defined shear stress; ii) establish adhesive interactions; iii) transmigrate across the endothelial layer; iv) interstitially migrate through the extracellular matrix.

All components of the device are sterilized for instance using 70% ethanol.

### Cell culture

Murine neutrophils are differentiated from myeloid progenitors immortalized with estrogen-regulated Hoxb8 (ER-Hoxb8), as previously described (Wang, Calvo et al. 2006). Mouse progenitors are maintained undifferentiated in Opti-MEM® (Life Technologies - Invitrogen) supplemented with 10 % heat-inactivated Fetal Bovine Serum (FBS; Euroclone), 1% penicillin/streptomycin (Life Technologies - Invitrogen), 30 uM beta-MercaptoEthanol (BME; Life Technologies - Invitrogen), 20 ng/mL Stem Cell Factor (SCF; PeproTech) and 1 uM beta-estradiol (estrogen; Sigma-Aldrich). Differentiation of myeloid progenitors into polymorphonuclear granulocytes is obtained by adding 20 ng/ml of Recombinant Human Granulocyte-Colony Stimulating Factor (rHuG-CSF; Myelostim - Italfarmaco) to the medium in the absence of estradiol.

Brain Endothelioma Cells (bEND.3; ATCC® Catalog No. CRL-2299) are coltured in Dulbecco's Modified Eagle's Medium (DMEM; Life Technologies - Invitrogen) supplemented with 15% FBS (Euroclone) and 1% penicillin/streptomycin (Life Technologies - Invitrogen).

Murine neutrophils used in *in vitro* functional assays are suspended in HBSS without Phenol Red, supplemented with 1 mM Ca²⁺/Mg²⁺, 10 mM Hepes and 0.1% BSA.

### Neutrophil Romanowski staining

Cells are stained with the "Diff-Quick" stain kit following manufacturer's instructions ("Diff-Quick" kit; IHC World)

### Flow cytometry

For flow cytometric analysis, murine neutrophils are labeled on ice with primary specific antibodies for 20 min (anti-mouse Gr-1, F4/80, CD117 antibodies are from AbD Serotec; anti-mouse CD11b is from BD Pharmingen^{™} - BD Biosciences; anti-mouse CXCR2 is from R&D Systems) followed by incubation with FITC-conjugated secondary antibodies for 30 min (Sigma-Aldrich). Cells are then washed and analyzed on a BD FACSCanto^{™} flow cytometer (BD Biosciences). Data analysis is performed with FlowJo Software (Tree Star, Inc)

### Neutrophil staining with fluorescent dyes

Murine neutrophils are harvested by centrifugation, resuspended in prewarmed CellTracker^{™} Green CMFDA or Orange CMRA (Molecular Probes - Invitrogen) solution (final concentration 0.5 uM in HBSS) and incubated for 20 minutes at 37°C in a water bath. The dye solution is replaced by complete colture medium and cells are incubated for additional 30 minutes at 37°C. Upon harvesting and washing by centrifugation, cells are resuspended in working medium at a concentration of 3 x 10⁶ cells/mL.

### Preparation of collagen I solution

Rat tail collagen I solution (1.6 mg/mL final concentration; BD Biosciences, Catalog Number 354249) is prepared following manufacturer's instructions using reagents kept at 4°C.

### Culture of endothelial cells onto the filter

The filter is coated for 90 minutes with 1.2 - 1.8 mg/mL Reduced Growth Factor Membrane Basement Matrix (Geltrex^{™}; GIBCO - Invitrogen). Endothelial cells (bEND.3 cells, 0.6 x 10⁶ cells) are then seeded onto the filter and cultured for two days in order to form a confluent monolayer. 20 hours prior to the assay, they are stimulated with 20 ng/mL Tumor Necrosis Factor alpha (TNF alpha; home-made recombinant pro-inflammatory cytokine, a kind gift from Angelo Corti)(Other pro-inflammatory chemokines - e. g. interleukin-1 (IL-1) - might be used). Finally, the endothelial cell monolayer is coated for 15 minutes at 37°C with 2 ug/mL KC (R&D Systems, Catalog Number 453-KC-050).

### In vitro assay for sequentially monitoring leukocyte extravasation and interstitial migration

Neutrophils labelled with either Green or Orange CellTracker^{™} dyes are suspended in working medium (3 x 10⁶ cells/mL) and perfused through the flow chamber at the desired shear stress. This might range between 1 and 5 dyn/cm², which is typical of post-capillary venules where leukocyte extravasation to inflamed tissues mainly occurs. The multistep leukocyte extravasation process is imaged bi-dimensionally for 4 minutes by focusing on the endothelial cell plane where leukocyte-endothelium interactions occur (such a time duration might vary depending on leukocyte subset and/or the inflammatory stimulus used in the assay). All cellular interactions with the substrate are determined by automated analysis with an in-house developed software (DedICATE - DynamIc Cell Adhesion Tracking software), however any equivalent software may be used. i) "Rolling"cells are defined as cells that roll for at least three cell diameters; ii) "transient" tethers are defined as cells attached briefly (<3 s) to the substrate; iii) "arrest" (firm) tethers are defined as tethered cells that immediately stop for at least 3 s. Frequencies of adhesive categories are determined as a percentage of cells flowing immediately over the substrate.

Once neutrophils have adhered onto and migrated across the endothelial layer, interstitial migration within the collagen matrix is imaged three-dimensionally for 30 minutes (such a time duration might vary depending on leukocyte subset and/or the inflammatory stimulus used in the assay). Time-lapse imaging of 25 z planes across the collagen gel is performed. Z stacks are acquired between a top plane, corresponding to the endothelial cell layer, and a bottom one, corresponding to a plane of the collagen matrix which is 100 um far from the top. Migrating cells are tracked manually with Volocity 3D Image Analysis Software to quantify: i) Track length (cell path), defined as accumulated distance; ii) Track vector (Displacement or Euclidean distance), defined as length of straight line between cell start and end point; iii) Speed, defined as velocity along the accumulated distance; iv) Directionality, defined as ratio between euclidean distance and accumulated distance (it gives indication about the directionality of the cell motility); v) Forward Migration Index (FMI), defined as ratio between displacement along a defined axis and accumulated distance (it represents the efficiency of forward migration of cells). Both leukocyte extravasation under flow and interstitial migration are conducted at 37°C.

### Video Acquisition

Real-time imaging of leukocyte trafficking is performed by using a spinning disk confocal microscope (set up for fluorescence imaging, objective 20X; PerkinElmer) equipped with a stage incubator and a motorized stage for 3D acquisition.

### Statistical analysis

Paired or unpaired two-tailed Student t test are calculated for comparison between groups. P values less than 0.05 are considered statistically significant.

Spearman's rank correlation coefficient is calculated for correlations between samples. P values less than 0.05 are considered to be statistically significant. Data are analyzed using Graphpad Prism (Graphpad Software, San Diego, CA)

### RESULTS

### Microfluidic chamber design (Fig. 1)

### Schematic view of the layers (synthetic)

The present flow chamber (Fig. 1a) consists of (from top to bottom, Fig. 1b):
- a top component (2), detailed in Fig. 1d, preferably made of steel, aluminum, rigid plastic or any non-flexible material;
- a lid, preferably made of plastic, preferably having a thickness from about 1 mm to about 1 cm, that should be compatible with the focal distance of the microscope. It should be transparent to allow imaging and robust. The lid has an inlet and an outlet port through which the cells enter and exit the channel (4, detailed in Fig. 1g);
- a gapped gasket (10), detailed in Fig. 1e, preferably made of silicone rubber and having a thickness from about 70 um to about 300 um, preferably about 200 um thick; the gasket is flexible to act as a gasket and is not porous;
- a filter (9) that is flexible, thin (15 um thick) porous (10⁵ pores/cm - ∅ 3-8 um). Filter pores are chosen based on the studied cell type, the filter is preferably made of a material transparent to fluorescent such as polycarbonate, the filter is optionally coated with cells, particularly endothelial cells;
- a gapped gasket (8), detailed in Fig. 1f, preferably made of silicone rubber, being flexible to act as a gasket, non-porous and having thickness from about 200 um to about 500 um, preferably about 500 um thick, with a channel-shaped gap where a three-dimensional extracellular matrix (e. g. collagen I matrix) and an underlying chemoattractant containing agarose solution are allowed to sequentially jellify;
- a slide (7), preferably made of transparent material such as glass, as thin as possible, generally from about 150 um to about 200 um, usually about 170 um;
- a bottom component (3), detailed in Fig. 1c, preferably made of steel, aluminum, rigid plastic or any non-flexible material.

### Detailed schematic view of the layers

The present flow chamber consists of a number of parts assembled in a multilayer configuration (Fig.1a) between two holders (2 and 3, preferably stainless steel) screwed together.

A polymeric and deformable gasket (10) is obtained with a rectangular shaped hole that has the role of a micro-channel (14)(Fig. 1e). This micro-channel is characterized by a flow section (wp

* hp) with a geometric design (high aspect ratio wp/hp) able to provide controlled flow conditions and defined wall shear stress on the surfaces of the channel.

The upper surface of the channel (14) is the bottom surface (17) of the (4) component (Fig. 1g). (4) is a transparent part, preferably polycarbonate, its bottom surface (17) is in contact with the gasket (10) while its upper surface shows two identical hose barbed connections (15a and 15b)(Fig. 1g). Inlet and outlet tubes (1 and 11) are connected to them (Fig. 1b). (15a) and (15b) show internal channels that pass through the component down to the gasket (10). Inlet and outlet are aligned to the channel (14) so that cell suspension such as leukocytes can flow from (1) to (11) passing along the micro-channel (14). (15a) and (15b) are located at the far ends of the micro-channel (14) in order to leave a proper channel length free from the fluid dynamic effects due to inlet and outlet ports and to make this portion of the channel transparent and observable with appropriated means such as an inverted microscope.

The bottom surface of the channel (14) is the upper surface of piece (9). (9) is a porous filter (Fig. 1b) characterized by small thickness and controlled porosity (about 50 to 200 pores/com, preferably about 105 pores/cm, the pores having a diameter between about 3 um to about 8 um). Each pore connects the upper micro-channel (14) to the bottom chamber (18) which is shaped in the gasket (8) (Fig. 1f). Endothelial cells can be coated on the upper surface of the filter (9), thereby resulting in a monolayer of cells on the bottom surface of the micro-channel (14).

The bottom surface of filter (9) lays on gasket (8), similar to gasket (10) but different for the thickness (Fig. 1b). The channel (18) is shaped in this gasket (8). On the bottom side, the cavity (20) is delimited by a slide (7)(Fig. 1b, preferably made of glass). The cavity (20) is filled with a porous gel matrix enabling interstitial migration of cells coming from the micro-channel (14) through the filter (9). Then, the filter (9) separates the flow channel (14) from the matrix chamber (20). The filler of the cavity (20) is made of two different gels, the one close to the microscope glass (7) functions as a reservoir for chemotactic factors. The position and the thickness of this part enable the formation of a chemotactic gradient across the upper part of the gel-chamber (20) up to the porous filter (9).

All these parts (4, 10, 9, 8, 7,) are assembled and kept in place by means of part (2) and part (3) (Fig. 1a and 1b). Part (2) and part (3) are screwed together by the threads (12 and 13)(Fig. 1c and Fig. 1d). O-rings (6 and 5) have the role to distribute the pressure exerted by part (2) and (3) on the multilayer assembly (4, 10, 9, 8, 7,), in order to avoid the leakage of fluid from the micro-channel (14) and chamber (18)(Fig. 1a and 1b). The feature (16) of the piece (4) (Fig. 1g) conforms with the cavity (19) of the piece (3) (Fig. 1c). The coupling of these two parts (6 + 19) avoid the dislocation of piece (4) during the assembly of the multilayer, especially when the O-ring (5) gets in contact with the upper surface of (4) while being (2) screwing on (3)(Fig. 1b).

### Experimental setup (Fig. 1h)

The above described assembled device models the *in vivo*-like 3D microenvironment where leukocytes traffic. Cells are allowed to enter through an inlet port (15a) and to flow along a channel (14) created by the lid (4) and the gapped gasket (10) over an endothelium-coated filter (9). The endothelial cells are primary cells or cell lines. The channel design and the flow create a controlled wall shear stress on the endothelial cell monolayer, which can be modified during the assay by operating on the flow rate generated by the pump, for instance a syringe pump able to control the fluid in the range of 10-2000 ul/min. Any pump able to control the fluid in the range of 10-2000 ul/min is suitable.

Moreover, endothelial cells are pre-treated with a pro-inflammatory cytokine, for instance TNF-alpha (20ng/ml) and pre-coated with a chemoattractant, such as KC in order to facilitate leukocyte arrest and subsequent transmigration. This portion of the chamber mimics the vascular milieu where the multistep leukocyte extravasation process occurs. The filter (9) lays over the jellified 3D collagen I-based matrix (1,6 mg/ml) where a chemoattractant (such as a chemokine), if present, can diffuse from an underlying agarose gel (1,2%) serving as chemoattractant reservoir. The presence of the chemoattractant is optional. When present, it increases cell motility and migration directionality. The agarose gel may be any gel in which the chemoattractant can diffuse towards the extracellular matrix and the filter. The gel should be transparent to fluorescence and needs to sustain the collagen layer.

This results in the formation of a concentration gradient via diffusion of the chemoattractant along the z-axis of the collagen matrix, which is perpendicular to the endothelial layer on top. Such collagen I-based and agarose gel layers are created sequentially into the channel-shaped gap (18) of the gasket (8) underlying the filter (9). The gasket (8) lays over the slide (7) located in the bottom part (3) of the device. This second portion of the chamber recapitulates the extravascular microenvironment where leukocyte 3D interstitial migration takes place (Fig.1h). The operating set up encompasses the above described device, suitably integrated in a system comprising: a pump (in particular a syringe pump) to generate the flow; a spinning disk confocal microscope (set up for fluorescence imaging) endowed with a stage incubator and a motorized stage for 3D imaging; a software for real-time acquisition and analysis of interstitial migration; a software tool for image processing and analysis of leukocyte-endothelial cell interactions (Fig. 1b)

In summary, in the present platform leukocytes are allowed to flow onto TNF-treated endothelial cells and establish a sequence of adhesive events (tethering, rolling, arrest) culminating with transmigration across the endothelium and interstitial migration within a 3D extracellular matrix. Alternatively cells may be treated with other pro-inflammatory cytokines such as IL-1. Further, cells may be treated with other different agents depending on the biological process to be studied

### Technical validation of the platform (Fig. 2)

The herein described microdevice aims at reconstituting a microenvironment characterized by the presence of a chemical gradient and of a shear flow that generates a controlled mechanical stimulus on cells in the flow chamber. The chemical gradient is generated from the agarose layer, which acts as a reservoir for chemokine: the latter diffuses through the collagen towards the flow chamber, where the receiving fluid flows along the porous filter (9), originating a controlled wall shear stress. In order to validate the presence of a controlled wall shear stress on the porous filter (9), its geometrical configuration was reproduced *in silico* after measuring (using the microscope motorized stage) the z coordinates of a number of points on the surface of the membrane. Three independent flow dynamic numerical simulations were performed in a flow chamber model incorporating the reconstructed surfaces. The contributions of the nonplanar features, explained by the soft consistency of the collagen matrix where the filter (9) is overlaid, were evaluated. Wall shear stress distribution was found to cover a range of values around the expected one, showing a variability from -30% to +8% on the most of the surface area, which confirms that a proper setting of the flow rate allows for the generation of a physiological wall shear stress. A color contour map of the wall shear stress on the filter is plotted as a function of the expected value T_{N} at a nominal flow rate in Fig. 2a. The generation and the persistence in time of a chemokine gradient across the collagen matrix were then estimated by a dedicated simulation representing a 3D portion of the microchannel; the simulation included the three compartments (agarose matrix, collagen gel and flow chamber) suitably connected by the filter (9) pores of the filter (9). The results of this simulation are shown at several time points up to 3600 s in Fig. 2b. At t=0 collagen and agarose are assumed to have the same chemokine concentration at the equilibrium, as the setting time of the matrixes - which took place prior to the assay - is long enough to reach this condition by diffusion, even if at the very beginning the agarose is the only matrix loaded with the chemokine. At t>0 the channel is filled by fluid flowing on the endothelial monolayer (not included in the simulation) which is very thin (5 - 7 um) and porous: the chemokine diffuses in the fluid through the adluminal pores, giving rise to a chemotactic gradient in the collagen layer. The concentration of chemokine in the collagen/agarose matrix decreases in time as the rate of the chemotactic gradient does, but the simulation shows that the present microdevice configuration involves the establishment and maintenance of a chemotactic gradient to guide the cells for the entire duration of the assay.

### In vitro modeling of the first key event in leukocyte trafficking during inflammation: the multistep extravasation process (Fig. 3)

The enabling potential of the device was demonstrated through a real-time *in vitro* reconstitution of leukocyte trafficking within vascular and extravascular *in vivo*-like microenvironments. The authors chose as cellular model primary mouse neutrophils differentiated from immortalized myeloid precursors (Wang, Calvo et al. 2006), as they play a key role in vascular inflammation (Phillipson and Kubes 2011; Kolaczkowska and Kubes 2013). Such neutrophils exhibited morphological differentiation, up-regulated typical surface differentiation markers including the neutrophil antigen Gr-1, the myeloid integrin Mac-1, the KC receptor CXCR-2 and, consistently, down-regulated the general myeloid trans-membrane receptor CD177 and the macrophage marker F4/80 (Fig. 3a).

In order to validate the biological usefulness of the platform, the authors first examined the multistep extravasation process of neutrophils flowing onto endothelial cells in the presence of physiological shear flow. To rule out the possibility that cell labeling with different cytosolic dyes could affect leukocyte behaviour, the authors compared wild-type neutrophils, labeled with either Green or Orange CellTracker^{™} dyes, for the ability to tether, roll, arrest onto and migrate across endothelial cells under shear flow (movies Fig. 3b and 3c). The acquired time lapse data were processed by an in-house developed software (DedICATE - DynamIc Cell Adhesion Tracking software), for automated tracking and analysis of leukocyte-endothelium interactions. Any other software may be used, for instance the one described in http://www.eng.buffalo.edu/∼neel/zhang_pplate.pdf. Within 2 minutes, the software is able to track the cells and output their adhesive category (transient adhesion, rolling, rolling and arrest, arrest for selected time), based on cell velocity. As expected, green- and red-labeled cells behaved similarly, with the majority of them establishing chemokine-induced arrest to the endothelium (Fig. 3d). Trans-endothelial migration also occurred (data not shown) as determined by manual scoring of cells whose fluorescence noticeably moved out of focus due to their movement underneath the endothelium, (movie Fig. 3c). Differential interference contrast imaging of the same microscopic field confirmed that fading fluorescence signals by individual arrested cells was a feature of transmigrating leukocytes.

### 4) In vitro modeling of the second key event in leukocyte trafficking during inflammation: interstitial migration (Fig.4)

The main advancement of the present device lies in the possibility of performing sequential imaging of leukocyte extravasation and the subsequent interstitial migration step. Indeed, once neutrophils in the present platform have adhered onto and migrated across the endothelial layer (Top layer in movie Fig 4a), their interstitial migration within the 3D collagen matrix (between top and bottom layers in movie Fig. 4a) can be imaged three-dimensionally and quantified (movie Fig. 4a). As for the multistep leukocyte extravasation process, green- and red-labeled wild-type neutrophils were compared for the ability to migrate in the x-, y-, z- axes through the collagen matrix where a gradient of KC (from bottom to top, along the z axis) was formed (movie Fig. 4a). To further dissect the mechanisms underlying 3D interstitial migration, that have only recently begun to emerge, the authors also assessed the leukocyte migratory behaviour in the absence of a chemotactic gradient (movie Fig. 4b). Compared to the absence of chemokine in the collagen matrix, in the presence of a gradient neutrophils migrated farther towards the chemoattractant source along the z-axis, as shown in Fig. 4c where the end time-point of the migration process in both conditions is depicted. Notably, 3D real-time imaging in the authors' set up allowed for a higher content, more sophisticated analysis of cell motility. Indeed, 3D manual cell tracking revealed that in the presence of a chemotactic gradient green and red neutrophils behaved similarly, were more motile (Fig. 4d) and showed higher displacement along the x-, y-, z-axes (Fig. 4e). As expected, neutrophils showed significantly increased speed, displacement and directionality when exposed to the chemokine and the migratory behaviour of green and red cells was comparable (Fig. 4f). Interestingly, a statistically significant correlation between KC-stimulated cell displacement and forward migration index (FMI) along the z-axis, but not along the x- and y-axes, emerged (Fig. 4g). Altogether these findings suggest that in the presence of a KC gradient within the collagen matrix, neutrophils are highly motile and migrate more efficiently in the direction of the gradient.

### References

- Abhyankar, V. V., M. W. Toepke, et al. (2008). "A platform for assessing chemotactic migration within a spatiotemporally defined 3D microenvironment." Lab on a chip 8(9): 1507-1515.
- Alon, R. and K. Ley (2008). "Cells on the run: shear-regulated integrin activation in leukocyte rolling and arrest on endothelial cells." Current opinion in cell biology 20(5): 525-532.
- Bianchi, E., R. Molteni, et al. (2013). "Microfluidics for in vitro biomimetic shear stress-dependent leukocyte adhesion assays." Journal of biomechanics 46(2): 276-283.
- Boyden, S. (1962). "The chemotactic effect of mixtures of antibody and antigen on polymorphonuclear leucocytes." The Journal of experimental medicine 115: 453-466.
- Brown, D. C. and R. S. Larson (2001). "Improvements to parallel plate flow chambers to reduce reagent and cellular requirements." BMC immunology 2: 9.
- Constantin, G., M. Majeed, et al. (2000). "Chemokines trigger immediate beta2 integrin affinity and mobility changes: differential regulation and roles in lymphocyte arrest under flow." Immunity 13(6): 759-769.
- Cooke, B. M., S. Usami, et al. (1993). "A simplified method for culture of endothelial cells and analysis of adhesion of blood cells under conditions of flow." Microvascular research 45(1): 33-45.
- Gerisch, G. and H. U. Keller (1981). "Chemotactic reorientation of granulocytes stimulated with micropipettes containing fMet-Leu-Phe." Journal of cell science 52: 1-10.
- Grabovsky, V., S. Feigelson, et al. (2000). "Subsecond induction of alpha4 integrin clustering by immobilized chemokines stimulates leukocyte tethering and rolling on endothelial vascular cell adhesion molecule 1 under flow conditions." J Exp Med 192(4): 495-506.
- Gunzer, M., A. Schafer, et al. (2000). "Antigen presentation in extracellular matrix: interactions of T cells with dendritic cells are dynamic, short lived, and sequential." Immunity 13(3): 323-332.
- Haessler, U., Y. Kalinin, et al. (2009). "An agarose-based microfluidic platform with a gradient buffer for 3D chemotaxis studies." Biomedical microdevices 11(4): 827-835.
- Han, S., J. J. Yan, et al. (2012). "A versatile assay for monitoring in vivo-like transendothelial migration ofneutrophils." Lab on a chip 12(20): 3861-3865.
- Hynes, R. O. (2002). "Integrins: bidirectional, allosteric signaling machines." Cell 110(6): 673-687.
- Irimia, D., S. Y. Liu, et al. (2006). "Microfluidic system for measuring neutrophil migratory responses to fast switches of chemical gradients." Lab on a chip 6(2): 191-198.
- Kinashi, T. (2005). "Intracellular signalling controlling integrin activation in lymphocytes." Nat Rev Immunol 5(7): 546-559.
- Kolaczkowska, E. and P. Kubes (2013). "Neutrophil recruitment and function in health and inflammation." Nature reviews. Immunolog 13(3): 159-175.
- Laudanna, C., J. Y. Kim, et al. (2002). "Rapid leukocyte integrin activation by chemokines." Immunol Rev 186: 37-46.
- Lawson, C., M. Rose, et al. (2010). "Leucocyte adhesion under haemodynamic flow conditions." Methods in molecular biology 616: 31-47.
- Ley, K., C. Laudanna, et al. (2007). "Getting to the site of inflammation: the leukocyte adhesion cascade updated." Nature reviews. Immunology 7(9): 678-689.
- Li Jeon, N., H. Baskaran, et al. (2002). "Neutrophil chemotaxis in linear and complex gradients of interleukin-8 formed in a microfabricated device." Nature biotechnology 20(8): 826-830.
- McEver, R. P. (2002). "Selectins: lectins that initiate cell adhesion under flow." Curr Opin Cell Biol 14(5): 581-586.
- McGettrick, H. M., A. Filer, et al. (2007). "Modulation of endothelial responses by the stromal microenvironment: effects on leucocyte recruitment." Biochemical Society transactions 35(Pt 5): 1161-1162.
- Molteni, R., C. L. Crespo, et al. (2009). "Beta-arrestin 2 is required for the induction and strengthening of integrin-mediated leukocyte adhesion during CXCR2-driven extravasation." Blood 114(5): 1073-1082.
- Molteni, R., M. Fabbri, et al. (2006). "Pathophysiology of leukocyte-tissue interactions." Current opinion in cell biology 18(5): 491-498.
- Nelson, R. D., P. G. Quie, et al. (1975). "Chemotaxis under agarose: a new and simple method for measuring chemotaxis and spontaneous migration of human polymorphonuclear leukocytes and monocytes." Journal of immunology 115(6): 1650-1656.
- Nourshargh, S., P. L. Hordijk, et al. (2010). "Breaching multiple barriers: leukocyte motility through venular walls and the interstitium." Nature reviews. Molecular cell biology 11(5): 366-378.
- Nourshargh, S. and F. M. Marelli-Berg (2005). "Transmigration through venular walls: a key regulator of leukocyte phenotype and function." Trends in immunology 26(3): 157-165.
- Phillipson, M. and P. Kubes (2011). "The neutrophil in vascular inflammation." Nature medicine 17(11): 1381-1390.
- Rabodzey, A., P. Alcaide, et al. (2008). "Mechanical forces induced by the transendothelial migration of human neutrophils." Biophysical journal 95(3): 1428-1438.
- Saadi, W., S. W. Rhee, et al. (2007). "Generation of stable concentration gradients in 2D and 3D environments using a microfluidic ladder chamber." Biomedical microdevices 9(5): 627-635.
- Sackmann, E. K., E. Berthier, et al. (2012). "Microfluidic kit-on-a-lid: a versatile platform for neutrophil chemotaxis assays." Blood 120(14): e45-53.
- Schreiber, T. H., V. Shinder, et al. (2007). "Shear flow-dependent integration of apical and subendothelial chemokines in T-cell transmigration: implications for locomotion and the multistep paradigm." Blood 109(4): 1381-1386.
- Sixt, M. and T. Lammermann (2011). "In vitro analysis of chemotactic leukocyte migration in 3D environments." Methods in molecular biology 769: 149-165.
- Wang, G. G., K. R. Calvo, et al. (2006). "Quantitative production of macrophages or neutrophils ex vivo using conditional Hoxb8." Nature methods 3(4): 287-293.
- Wolf, K., S. Alexander, et al. (2009). "Collagen-based cell migration models in vitro and in vivo." Seminars in cell & developmental biology 20(8): 931-941.
- Zicha, D., G. A. Dunn, et al. (1991). "A new direct-viewing chemotaxis chamber." Journal of cell science 99 ( Pt 4): 769-775.
- Zigmond, S. H. (1977). "Ability of polymorphonuclear leukocytes to orient in gradients of chemotactic factors." The Journal of cell biology 75(2 Pt 1): 606-616.

## Claims

1. A flow chamber for cell assay essentially comprising :
- a bottom holder (3) and a top holder (2), both holders having each one at least one
opening, the bottom and the top holder being sealable to each other;
said holders containing from the top to the bottom:
- a lid (4) comprising two connections (15a) and (15b), passing through the at least one opening of the top holder (2), one connectable to an inlet port (1) and the other connectable to an outlet port (11);
- a first gasket (10) comprising a first channel (14), each far end of the channel (14) being aligned with the connections (15a) and (15b), respectively;
- a filter (9), optionally coated with cells;
- a second gasket (8) comprising a second channel (18) being aligned with the first channel (14) and having at least the same area as said first channel (14);
- a slide (7), so that the second channel (18) and the slide (7) define a chamber (20) being filled with at least one layer of gel.

2. The flow chamber according to claim 1 wherein the top holder (2) and the lid (4) are assembled in one piece.

3. The flow chamber according to claim 1 or 2 wherein the cells on filter (9) are endothelial cells.

4. The flow chamber according to claim 3 wherein the endothelial cells are stimulated with a pro-inflammatory agent, preferably TNF or IL-1.

5. The flow chamber according to claim 3 or 4 wherein the endothelial cells are coated with an agent that increases cell arrest and transmigration across endothelial cells.

6. The flow chamber according to claim 5 wherein the agent is a chemoattractant, preferably the chemoattractant is selected from the group consisting of: formyl peptides, chemokines (mostly CC or CXC chemokines), complement 3a (C3a) and complement (C5a), leukotrienes.

7. The flow chamber according to any one of previous claims wherein the chamber (20) is filled with a first and a second layer of gel, the second layer of gel optionally comprising an agent that increases cell motility, preferably a chemoattractant, more preferably the chemoattractant is selected from the group consisting of: formyl peptides, chemokines (mostly CC or CXC chemokines), complement 3a (C3a) and complement (C5a), leukotrienes.

8. A device for measuring cell adhesion and/or transendothelial migration and/or interstitial migration comprising the flow chamber according to any one of previous claims, and a flow producing means connected thereto through the inlet port (1).

9. Use of the flow chamber according to any one of claim 1 to 7 or the device according to claim 8 to assess adhesion and/or transendothelial migration and/or interstitial migration of cells introduced by the inlet port (1).

10. A method to assess cell adhesion to endothelial cells by means of the flow chamber according to any one of claims 1 to 7 or the device according to claim 8 comprising the steps of:
- introducing a cell suspension through the inlet port (1) and allowing the cells to flow through the first connector (15a), the channel (14) and to adhere to the filter (9) coated with endothelial cells;
- measuring with appropriate imaging means the adhesion of cells to endothelial cells.

11. A method to assess cell transmigration across endothelial cells by means of the flow chamber according to any one of claims 1 to 7 or the device according to claim 8 comprising the steps of:
- introducing a cell suspension through the inlet port (1) and allowing the cells to flow through the first connector (15a), the channel (14), to adhere to and migrate across the filter (9) coated with endothelial cells to reach the chamber (20);
- measuring with appropriate imaging means the transendothelial migration.

12. A method to assess cell interstitial migration by means of the flow chamber according to any one of claims 1 to 7 or the device according to claim 8 comprising the steps of:
- introducing a cell suspension through the inlet port (1) and allowing the cells to flow through the first connector 15(a), the channel (14), to adhere to and migrate across the filter (9) optionally coated with endothelial cells into the chamber (20) filled with the first and second gel layers, to migrate through the first gel layer, laid over the second one, said second gel layer optionally comprising an agent that increases cell motility;
- measuring with appropriate imaging means the interstitial migration of cells.

13. A method to assess cell adhesion to endothelial cells, cell transmigration across endothelial cells and cell interstitial migration or cell adhesion to and transmigration across endothelial cells or cell transmigration across endothelial cells and interstitial migration by means of the flow chamber according to any one of claims 1 to 7 or the device according to claim 8 comprising the step of:
- introducing a cell suspension through the inlet port (1) and allowing the cells to flow through the first connector (15a), the channel (14), to adhere to and migrate across the filter (9) coated with endothelial cells into the chamber (20) filled the first and second gel layers, and to migrate through the first gel layer, laid over the second one, said second gel layer optionally comprising an agent that increases cell motility;
- measuring with appropriate imaging means the adhesion of cells to endothelial cells, cell transmigration across endothelial cells and the interstitial migration of cells.

14. The method according to any one of claims 10 to 12 wherein the cell suspension is a leukocyte suspension, a tumor cell suspension or a stem cell suspension.

15. A method to identify an agent inhibiting extravasation and/or interstitial migration comprising using the flow chamber according to any one of claim 1 to 7 or the device of claim 8 in the presence or absence of a test agent, wherein if decreased migration and/or adhesion is observed in the presence of the test agent when compared to migration and/or adhesion in absence of the test agent, then the inhibitory agent is identified.
